# EUROPÄISCHE PATENTSCHRIFT

(11) **EP 3 118 968 B1**
(45) Veröffentlichungstag und Bekanntmachung des Hinweises auf die Patenterteilung: **21.11.2018**
(21) Anmeldenummer: 16001578.0
(22) Anmeldetag: 15.07.2016
(51) Int. Cl.: H02J 9/06, A61M 16/00

(54) **VORRICHTUNG UND VERFAHREN ZUR OPTIMIERUNG DES WÄRMEHAUSHALTS IN BEATMUNGSGERÄTEN MIT GEBLÄSEMOTOR**
DEVICE AND METHOD FOR OPTIMISING THE INTERNAL HEAT IN VENTILATION DEVICES WITH BLOWER MOTOR
DISPOSITIF ET PROCEDE D'OPTIMISATION DU BILAN THERMIQUE DANS DES APPAREILS RESPIRATOIRES AYANT UN MOTEUR DE VENTILATEUR

(30) Priorität: 16.07.2015 DE 102015009080
(43) Veröffentlichungstag der Anmeldung: 18.01.2017
(73) Patentinhaber: Löwenstein Medical Technology S.A., 2557 Luxembourg (LU)
(72) Erfinder: Houillon, Thomas, D-21720 Grünendeich (DE)
(74) Vertreter: Marx, Thomas

(56) Entgegenhaltungen:
- EP-A1- 2 445 082
- EP-A1- 2 612 688
- US-A1- 2012 055 480
- US-A1- 2014 366 876
- US-A1- 2015 180 276

## Beschreibung

Bei Beatmungsgeräten mit dynamischen Gebläse-/Turbinenmotoren treten bei der Beschleunigung während der Inspiration kurzzeitig elektrische Leistungsspitzen auf, die typisch oberhalb der zulässigen Dauerlast des Versorgungsnetzteils liegen können. Die Lastspitzen können zudem auch oberhalb des spezifizierten Bereichs für tolerierte Lastspitzen des Versorgungsnetzteils liegen. Diese Leistungsspitzen erzeugen eine erhöhte Wärmeentwicklung am Versorgungsnetzteil selbst und in dessen Umgebung innerhalb des Gerätes.

Die EP2612688 (A1) betrifft ein Beatmungsgerät, bei dem die Bremsenergie des Elektromotors zumindest phasenweise in einer Spannungssenke, beispielsweise in Form eines Kondensators gespeichert wird. Zum Beschleunigen des Elektromotors wird die Energie wieder dem Kondensator entnommen. Zentrales Element der Leistungsschaltung der EP2612688 (A1) ist ein Wandler. Durch Verwendung eines DC/DC-Synchron-Wandlers wird ein dauerhafter bidirektionaler Stromfluss zwischen Motor und Kondensator ermöglicht.

Die US2014366876 (A1) betrifft ein Beatmungsgerät mit verbesserter Leistungsversorgung. Die US2014366876 (A1) schlägt vor, dass eine energiesparende Beatmungsstrategie angewandt wird, wenn sensorisch festgestellt ist, dass die Spannungsquelle zu heiß wird.

Die EP2445082 (A1) betrifft ein Beatmungsgerät mit einer umschaltbaren Leistungsversorgung aus einem Netzteil oder einer internen Batterie oder externen Batterien. Gemäß der EP2445082 (A1) werden die unterschiedlichen Energiequellen genutzt, um eine kontinuierliche Leistungsversorgung zu gewährleisten.

Die US2015180276 (A1) offenbart eine Methode der unterbrechungsfreien Energieversorgung eines Power-over-Ethernet (PoE) Gerätes.

Die US2012055480 (A1) offenbart einen Sauerstoffkonzentrator mit einer Energieversorgung.

Es besteht daher ein Bedarf, die Wärmeentwicklung zu reduzieren.

Aufgabe der Erfindung ist daher eine Leistungsschaltung und ein Verfahren zur Leistungsschaltung für ein Beatmungsgerät anzugeben, welche die Wärmeentwicklung am Versorgungsnetzteil minimieren und die Lastspitzen bedarfsweise abpuffern.

Diese Aufgabe wird durch die erfindungsgemäßen Merkmale der Ansprüche 1 und 10 gelöst.

Die Leistungsschaltung ist auch dadurch gekennzeichnet, dass die primäre Energiequelle, die als Netzteil ausgeführt ist, über den Akkulader den Akkumulator, als die sekundäre Energiequelle, versorgt.

Die Leistungsschaltung ist alternativ oder ergänzend auch dadurch gekennzeichnet, dass ein Leistungskontroller und eine Strommessung zwischengeschaltet sind und die aktuell verbrauchte Leistung gemessen wird.

Die Leistungsschaltung ist alternativ oder ergänzend auch dadurch gekennzeichnet, dass der von der Strommessung gemessene Wert der verbrauchten Leistung durch einen Komparator ausgewertet wird.

Die Leistungsschaltung ist alternativ oder ergänzend auch dadurch gekennzeichnet, dass der Komparator eine integrierte Hysterese aufweist, wobei erfindungsgemäss der Komparator immer dann auf Versorgung durch die sekundäre Energiequelle um-/zugeschaltet, wenn die verbrauchte Leistung oberhalb der im Komparator eingestellten Schwelle liegt, zuzüglich einer Abfallzeit bei Hysterese.

Die Leistungsschaltung ist alternativ oder ergänzend auch dadurch gekennzeichnet, dass der Komparator die Akkuladung ein- und ausschaltet.

Die Leistungsschaltung ist alternativ oder ergänzend auch dadurch gekennzeichnet, dass zusätzlich ein Anschluss vorgesehen ist der zusätzlich oder alternativ zur primären Energiequelle und zur sekundären Energiequelle die elektrischen Abnehmer und das Beatmungsgerät bei einem mobilen Einsatz des Beatmungsgerätes versorgt.

Die Leistungsschaltung ist alternativ oder ergänzend auch dadurch gekennzeichnet, dass der Mikrocontroller die Regelfunktion durch Ansteuerung des Akkuladers und des Leistungskontrollers übernimmt.

Die Leistungsschaltung ist alternativ oder ergänzend auch dadurch gekennzeichnet, dass die Schaltung in Hardware realisiert ist und zusätzlich zumindest teilweise in Firmware realisiert ist.

Die Leistungsschaltung ist alternativ oder ergänzend auch dadurch gekennzeichnet, dass die Schaltung in Hardware realisiert ist und zusätzlich in Firmware realisiert ist, wobei die Schaltung standardmäßig in Firmware ausgeführt wird und lediglich bei Ausfall oder Störung der Firmware, die Hardware-Schaltung verwendet wird.

Das Verfahren ist alternativ oder ergänzend auch dadurch gekennzeichnet, dass die primäre Energiequelle, die als Netzteil ausgeführt ist, über den Akkulader den Akkumulator, als die sekundäre Energiequelle, versorgt.

Das Verfahren ist alternativ oder ergänzend auch dadurch gekennzeichnet, dass ein Leistungskontroller und eine Strommessung zwischengeschaltet sind und die aktuell verbrauchte Leistung gemessen wird.

Das Verfahren ist alternativ oder ergänzend auch dadurch gekennzeichnet, dass der von der Strommessung gemessene Wert der verbrauchten Leistung durch einen- Komparator oder einen Mikrokontroller ausgewertet wird.

Das Verfahren ist alternativ oder ergänzend auch dadurch gekennzeichnet, dass der Komparator oder der Mikrokontroller eine integrierte Hysterese aufweist, wobei erfindungsgemäss der Komparator oder der Mikrokontroller immer dann auf Versorgung durch die sekundäre Energiequelle um-/zugeschaltet, wenn die verbrauchte Leistung oberhalb der im Komparator oder Mikrokontroller eingestellten Schwelle liegt, zuzüglich einer Abfallzeit bei Hysterese.

Das Verfahren ist alternativ oder ergänzend auch dadurch gekennzeichnet, dass oder der Mikrokontroller die Regelfunktion durch Ansteuerung des Akkuladers und des Leistungskontrollers übernimmt.

Das Verfahren ist alternativ oder ergänzend auch dadurch gekennzeichnet, dass Schaltung in Hardware realisiert ist und zusätzlich in Firmware realisiert ist, wobei die die Schaltung standardmäßig in Firmware ausgeführt wird und lediglich bei Ausfall oder Störung der Firmware, die Hardware-Schaltung verwendet wird.

Bei Geräten mit integriertem Akkumulator können diese Leistungsspitzen durch kurzzeitiges Zuschalten des Akkumulators unterstützt werden und so das Netzteil deutlich entlasten. Ebenso kann kurzzeitig von Netzteilversorgung auf Akkumulatorversorgung umgeschaltet werden. Zu diesem Zweck wird eine elektronische Schaltung eingefügt, die entweder selbstständig (frei von Software) in der Lage ist, die aktuelle Leistung im Gerät zu überwachen und den Akkumulator zuzuschalten und/oder dies softwaregestützt tut. Durch diese Maßnahme wird die Erwärmung des Versorgungsnetzteils im Gerät stark reduziert. Eine geringere Erwärmung des Versorgungsnetzteils trägt auch zur Verlängerung der Lebensdauer desselben bei, da auch im Netzteil selbst Elektrolytkondensatoren verbaut sind. Diese trocknen bei höheren Temperaturbedingungen früher aus. Ergänzend können die Leistungsspitzen auch von einem, oder mehreren Kondensator/en so abgemildert werden, dass sie innerhalb des Bereichs für kurzzeitige Lastspitzen des Versorgungsnetzteils bleiben.

Durch Einfügen von einem, oder mehrerer Kondensator/en können diese Spitzen so abgemildert werden, dass sie innerhalb des Bereichs für kurzzeitige Lastspitzen des Versorgungsnetzteils bleiben.

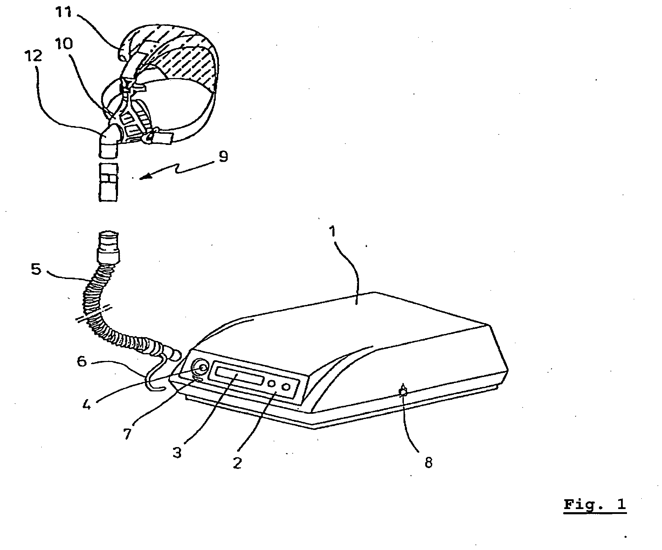

Fig. 1 zeigt den grundsätzlichen Aufbau einer Vorrichtung zur Beatmung. Im Bereich des Beatmungsgerätes (1) mit Bedienfeld (2) sowie Anzeige (3) ist in einem Geräteinnenraum ein Elektromotor (20, nicht dargestellt) mit Gebläse angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Im Bereich einer dem Gerätegehäuse (1) abgewandten Ausdehnung des Verbindungsschlauches (5) kann ein Ausatmungselement (9) angeordnet sein. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Der Motor des Beatmungsgerätes kann als ein mehrphasiger Motor realisiert sein. Als Motor können bürsten- und sensorlose Gleichstrommotoren, sowie Synchronmotoren, verwendet werden. Im Bereich des Gerätegehäuses befindet sich ein Bedienfeld für die Anwenderinformation und / oder Anwendersteuerung. Bevorzugt ist das Beatmungsgerät tragbar und verfügt optional über eine Energieversorgung, die die Beatmung zumindest für mehr als 2 Stunden aufrechterhält, wenn das Gerät nicht über einen Netzstecker mit Energie versorgt wird. Die Mobilität ist durch die zusätzliche oder optionale Anschlussmöglichkeit an das Bordnetz eines Fahrzeugs (über 12 oder 24 Volt) oder an eine entsprechende andere Energieversorgung erweiterbar.

In einem Geräteinnenraum ist eine Atemgaspumpe angeordnet, die als Elektro-Motor mit Lüfterrad ausgeführt ist, dessen Betrieb über eine Motorsteuerung regelbar ist. Der Betrieb des Motors und dessen Leistungsregelung sind durch die Motorsteuerung regelbar. Die Motorsteuerung berücksichtigt Daten von zumindest einer Sensoreinrichtung. Die Sensoreinrichtung ermittelt zumindest ein mit dem Atemgasstrom in Zusammenhang stehendes Signal. Ein Analysator ermittelt aus dem mit dem Atemgasstrom in Zusammenhang stehenden Signal beispielsweise Inspirationsphasen und Exspirationsphasen. In zumindest einem Betriebszustand regelt die Motorsteuerung die Lüfterraddrehzahl in Abhängigkeit der ermittelten Atemphase derart, dass während der Inspirationsphase ein im Wesentlichen konstanter positiven Druck aufrechterhalten wird. Der Motor ist derart ausgelegt, dass durch Drehzahländerung ein Druckbereich von beispielsweise im Wesentlichen 0 bis 80 mbar einstellbar ist. Druckänderungen werden durch Drehzahländerungen des Lüfterrades realisiert.

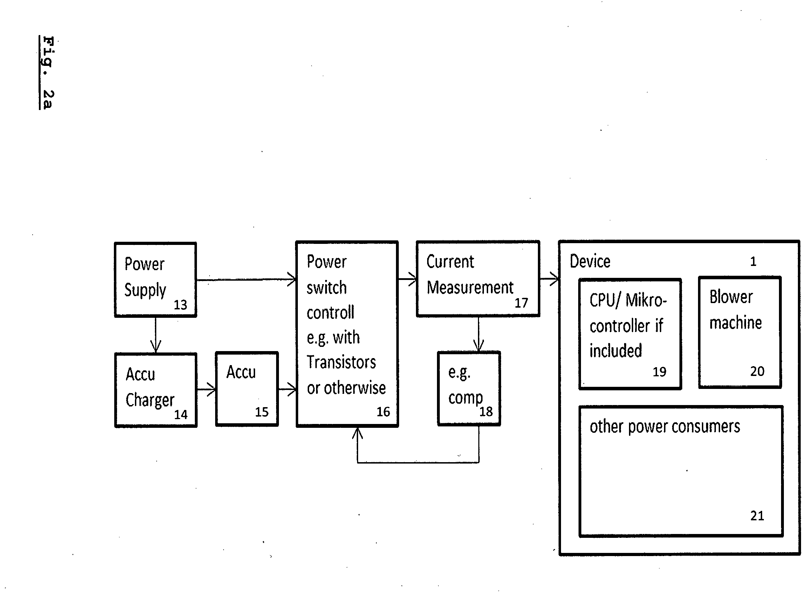

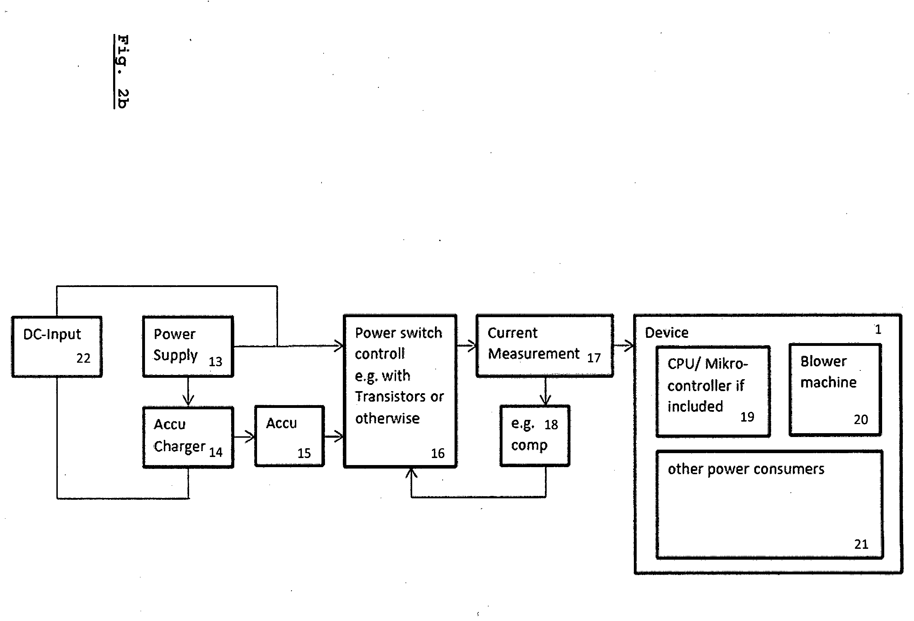

In Fig. 2a und 2b ist die hardwarebasierte Umschaltung schematisch dargestellt.

Aus der Energiequelle (13), die als Netzteil (13) ausgeführt sein kann, wird über den Akkulader (14) der Akku (15) geladen. Die primäre Energiequelle (13) kann alternativ oder ergänzend als interne Versorgung, beispielsweise in Form eines Netzteiles welches im Gerätegehäuse angeordnet ist, oder als externe Versorgung, beispielsweise in Form eines Netzteiles welches außerhalb des Gerätegehäuses angeordnet ist, ausgeführt sein. Die primäre Energiequelle kann alternativ oder ergänzend als externe Versorgung, beispielsweise in Form eines Netzteiles oder als Akkumulator (eines Rollstuhles) oder als Energieversorgung (Akkumulator oder Batterie) eines Fahrzeuges (PKW, Bus, Flugzeug) ausgebildet sein und beispielsweise 6 - 10 V leisten oder 10 - 50 V leisten oder 50 - 360 V leisten.

Zudem versorgt die Energiequelle insbesondere das Gerät (1), mit Mikrocontroller (19), Motor (20) und weiteren elektrischen Verbrauchern (21). Zwischengeschaltet ist ein Leistungskontroller (16) und eine Strommessung (17). Die aktuell verbrauchte Leistung wird permanent gemessen (current measurement, 17). Der gemessene Wert kann z.B. durch einen Komparator (comp, 18) ausgewertet werden. Wird ein Komparator (18) verwendet, so kann man an diesem einstellen, wie hoch die Schwelle sein soll, ab der umgeschaltet/zugeschaltet wird. Über einen Komparator (18) mit integrierter Hysterese kann eine Nachlaufzeit für das Rückschalten zum Normalbetrieb eingestellt werden. Bei dieser Umsetzung ist immer dann um-/zugeschaltet,' wenn die verbrauchte Leistung oberhalb der im Komparator (18) eingestellten Schwelle liegt, zzgl. einer Abfallzeit bei Hysterese. Der Komparator (18) ist im Sinne dieser Erfindung jede elektronische Schaltung oder jedes Bauteil, welches zwei Spannungen vergleich oder die Aufgaben eines herkömmlichen Komparators äquivalent löst. Beispielsweise sind dies eine BiPolar Transistorschaltung mit Strombegrenzung oder eine Transistorschaltung oder ein Operationsverstärker oder eine ADC-Wandlung mit boolescher Logik oder ein PTC.

Die Akkuladung soll hier nicht weiter betrachtet werden. Denkbar ist aber, dass der Komparator (18) zusätzlich zur Zu-/Umschaltung Vize-Versa die Akkuladung (Accu Charger, 14) ein- und ausschaltet. Ebenso gut kann diese auch vollständig autark den Akkumulator (15) je nach seinen Bedürfnissen laden.

In Figur 2b ist der Aufbau mit dem Anschluss (22) dargestellt. Der Anschluss (22) kann beispielsweise ein Anschluss für ein zusätzliches Netzteil und/oder ein Anschluss für eine externe Versorgung aus einem elektrischen Rollstuhl und/oder Anschluss für eine externe Versorgung aus einem Fahrzeug dargestellt. Der Anschluss versorgt hier zusätzlich oder alternativ zur primären Energiequelle (13) die Schaltung und das Beatmungsgerät (1) beispielsweise bei einem mobilen Einsatz des Beatmungsgerätes auf einem Rollstuhl oder in einem Fahrzeug. Der Anschluss kann zusätzlich oder alternativ zur primären Energiequelle (13) und zur sekundären Energiequelle (15) die elektrischen Abnehmer und das Beatmungsgerät (1) bei einem mobilen Einsatz des Beatmungsgerätes versorgen.

Das Beatmungsgerät ist dadurch über die Energiequelle (13) für den Heimbereich zur Versorgung mit üblicher Netzspannung aus der . Steckdose beispielsweise 110 V, 220 V, 230 V ausgelegt. Das Beatmungsgerät ist zusätzlich für den mobilen Einsatz über den Akkumulator (15) zur Versorgung mit üblicher Spannung wie beispielsweise mit 12V, 24 V, 48 V ausgelegt. Der interne Akku (15) ermöglicht die Aufrechterhaltung der Beatmung für beispielsweise 4 - 12 Stunden. Als Sicherheitsfunktion, wenn der interne Akku (15) leer ist oder nicht funktionsfähig, weist das Beatmungsgerät zusätzlich für den mobilen Einsatz den Anschluss (22) zur Versorgung mit üblicher Spannung aus einem Rollstuhl (Akkumulator, Batterie) oder einem Fahrzeug (Akkumulator, Batterie) oder einer anderen mobilen Quelle wie Akkumulator, Batterie beispielsweise mit 12V, 24 V, 48 V auf. Über den Anschluss (22) wird beispielsweise der Lader (14) versorgt, damit der Akkumulator geladen werden kann und ein Betrieb des Gerätes möglich ist. Der Anschluss (22) kann hier auch alternativ oder ergänzend den Leistungskontroller (16) versorgen und den Mikrokontroller (19) und das Gebläse (20) und weitere Abnehmer ein Betrieb des Gerätes möglich ist.

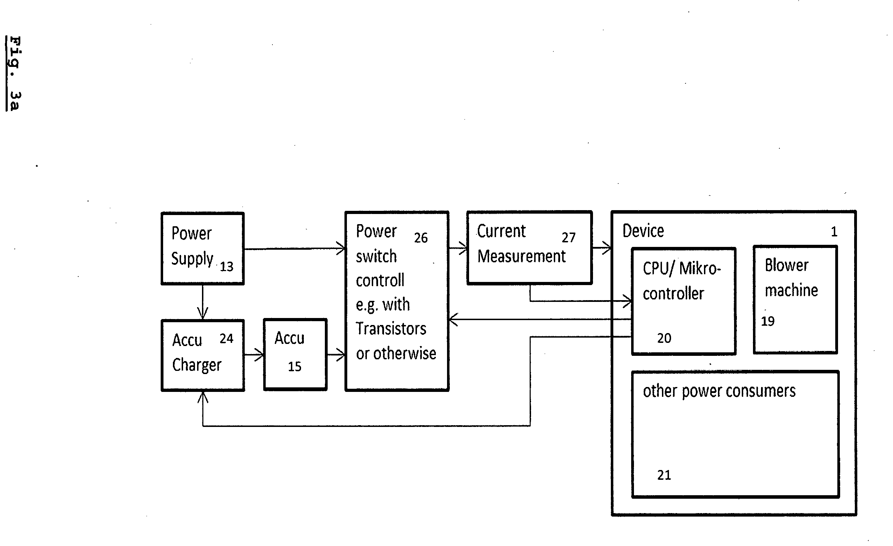

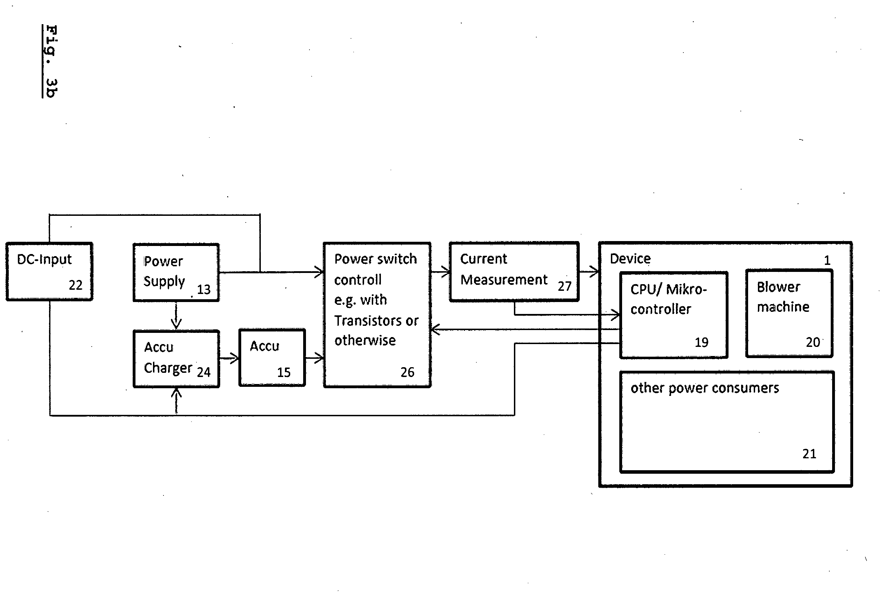

In Fig. 3a und 3b ist die softwarebasierte Umschaltung schematisch dargestellt.

Der Grundaufbau der Schaltung entspricht dem in Fig. 2 gezeigten Aufbau. Allerdings übernimmt hier der Mikrocontroller (19) die Regelfunktion durch Ansteuerung des Akkuladers (24) und des Leistungskontrollers (26). Die Strommesswerte werden durch die Strommessung (27) an den Mikrokontroller geliefert. Die aktuell verbrauchte Leistung wird permanent gemessen (current measurement, 27). Der gemessene Wert wird beispielhaft durch einen Analogeingang am Mikrocontroller (19) aufgenommen und weiterverarbeitet. Der Softwareentwickler hat hier nun alle Freiheiten die Schwelle für die Um-/Zuschaltung frei, dynamisch und zeitgesteuert zu programmieren. Es kann beispielsweise an der Steilheit einer Flanke erkannt werden, dass sich aktuell eine Spitze aufbaut und dadurch schon vor Verlassen des Dauerlastbereichs des Versorgungsnetzteils um-/zugeschaltet werden. Der Wärmehaushalt kann so noch effektiver reduziert werden. Über den Mikrocontroller (19) können mit dieser Umsetzung auch die Ladezeiten des Akkuladers sehr sinnhaft und effektiv an die Zu-/Umschaltung angepasst werden. In Figur 3b ist der Aufbau mit dem Anschluss (22) dargestellt. Der Anschluss (22) kann beispielsweise ein Anschluss für ein zusätzliches Netzteil und/oder ein Anschluss für eine externe Versorgung aus einem elektrischen Rollstuhl und/oder Anschluss für eine externe Versorgung aus einem Fahrzeug dargestellt. Der Anschluss kann zusätzlich oder alternativ zur primären Energiequelle (13) und zur sekundären Energiequelle (15) die elektrischen Abnehmer und das Beatmungsgerät (1) bei einem mobilen Einsatz des Beatmungsgerätes versorgen. Der Anschluss versorgt beispielsweise zusätzlich oder alternativ zur primären Energiequelle (13) die Schaltung und das Beatmungsgerät (1) beispielsweise bei einem mobilen Einsatz des Beatmungsgerätes auf einem Rollstuhl oder in einem Fahrzeug. Das Beatmungsgerät ist dadurch über die Energiequelle (13) für den Heimbereich zur Versorgung mit üblicher Netzspannung aus der Steckdose beispielsweise 110 V, 220 V, 230 V ausgelegt. Das Beatmungsgerät ist zusätzlich für den mobilen Einsatz über den Akkumulator (15) zur Versorgung mit üblicher Spannung wie beispielsweise mit 12V, 24 V, 48 V ausgelegt. Der interne Akku (15) ermöglicht die Aufrechterhaltung der Beatmung für beispielsweise 4 - 12 Stunden. Als Sicherheitsfunktion, wenn der interne Akku (15) leer ist oder nicht funktionsfähig, weist das Beatmungsgerät zusätzlich für den mobilen Einsatz den Anschluss (22) zur Versorgung mit üblicher Spannung aus einem Rollstuhl (Akkumulator, Batterie) oder einem Fahrzeug (Akkumulator, Batterie) oder einer anderen mobilen Quelle wie Akkumulator, Batterie beispielsweise mit 12V, 24 V, 48 V auf. Über den Anschluss (22) wird beispielsweise der Lader (24) versorgt, damit der Akkumulator geladen werden kann und ein Betrieb des Gerätes möglich ist. Der Anschluss (22) kann hier auch alternativ oder ergänzend den Leistungskontroller (26) versorgen und den Mikrokontroller (19) und das Gebläse (20) und weitere Abnehmer ein Betrieb des Gerätes möglich ist.

## Patentansprüche

1. Leistungsschaltung für ein Beatmungsgerät (1) mit Elektromotor (20) und einer primären Energiequelle (13) zur Versorgung zumindest eines elektrischen Abnehmers (16, 17, 18, 19, 20, 21, 26, 27) und einer sekundären Energiequelle (15) zur zumindest zeitweisen Versorgung des elektrischen Abnehmers (16, 17, 18, 19, 20, 21, 26, 27) **dadurch gekennzeichnet, dass** eine aktuell verbrauchte Leistung gemessen (16, 17) und ausgewertet wird (18, 19) und ein Komparator (18, 19) einen Schwellenwert für die aktuell verbrauchte Leistung aufweist wobei der Komparator (18, 19), abhängig vom Erreichen des Schwellenwertes, einem Leistungskontroller (16, 26) signalisiert, die Leistung für die Versorgung des elektrischen Abnehmers (16, 17, 18, 19, 20, 21, 26, 27) zumindest teilweise der sekundären Energiequelle (15) zu entnehmen, wobei immer dann auf Versorgung durch die sekundäre Energiequelle um/zugeschaltet wird, wenn die verbrauchte Leistung oberhalb der im Komparator (18, 19) eingestellten Schwelle liegt.

2. Leistungsschaltung nach Anspruch 1 **dadurch gekennzeichnet, dass** die primäre Energiequelle (13), die als Netzteil (13) ausgeführt ist, über einen Akkulader (14) einen Akkumulator (15), als die sekundäre Energiequelle (15), versorgt.

3. Leistungsschaltung nach Anspruch 1 oder 2 **dadurch gekennzeichnet, dass** ein Leistungskontroller (16) und eine Strommessung (17) zwischengeschaltet sind und die aktuell verbrauchte Leistung gemessen wird.

4. Leistungsschaltung nach zumindest einem der vorhergehenden Ansprüche **dadurch gekennzeichnet, dass** der von der Strommessung (17) gemessene Wert der verbrauchten Leistung durch den Komparator (18) ausgewertet wird.

5. Leistungsschaltung nach zumindest einem der vorhergehenden Ansprüche **dadurch gekennzeichnet, dass** der Komparator (18) eine integrierte Hysterese aufweist, wobei der Komparator immer dann auf die Versorgung durch die sekundäre Energiequelle (15) um/ zuschaltet, wenn die verbrauchte Leistung oberhalb der im Komparator (18) eingestellten Schwelle liegt, zuzüglich einer Abfallzeit bei Hysterese.

6. Leistungsschaltung nach zumindest einem der vorhergehenden Ansprüche **dadurch gekennzeichnet, dass** der Komparator (18) eine Akkuladung (Accu Charger, 14) ein- und ausschaltet.

7. Leistungsschaltung nach zumindest einem der vorhergehenden Ansprüche **dadurch gekennzeichnet, dass** zusätzlich ein Anschluss (22) vorgesehen ist der zusätzlich oder alternativ zur primären Energiequelle (13) und zur sekundären Energiequelle (15) die elektrischen Abnehmer und das Beatmungsgerät (1) bei einem mobilen Einsatz des Beatmungsgerätes versorgt.

8. Leistungsschaltung nach zumindest einem der vorhergehenden Ansprüche **dadurch gekennzeichnet, dass** ein Mikrocontroller (19) die Regelfunktion durch Ansteuerung des Akkuladers (24) und des Leistungskontrollers (26) übernimmt.

9. Leistungsschaltung nach zumindest einem der vorhergehenden Ansprüche **dadurch gekennzeichnet, dass** die Schaltung in Hardware realisiert ist und zusätzlich in Firmware realisiert ist.

10. Verfahren zur Leistungsschaltung bei einem Beatmungsgerät (1) mit Elektromotor (20) und einer primären Energiequelle (13) zur Versorgung zumindest eines elektrischen Abnehmers (16, 17, 18, 19, 20, 21, 26, 27) und einer sekundären Energiequelle (15) zur zumindest zeitweisen Versorgung des elektrischen Abnehmers (16, 17, 18, 19, 20, 21, 26, 27) **dadurch gekennzeichnet, dass** eine aktuell verbrauchte Leistung gemessen (16, 17) und ausgewertet wird (18, 19) und ein Komparator (18, 19) einen Schwellenwert für die aktuell verbrauchte Leistung aufweist wobei der Komparator (18, 19), abhängig vom Erreichen des Schwellenwertes, einem Leistungskontroller (16, 26) signalisiert, die Leistung für die Versorgung des elektrischen Abnehmers (16, 17, 18, 19, 20, 21, 26, 27) zumindest teilweise der sekundären Energiequelle (15) zu entnehmen, wobei immer dann auf Versorgung durch die sekundäre Energiequelle um-/zugeschaltet wird, wenn die verbrauchte Leistung oberhalb der im Komparator (18, 19) eingestellten Schwelle liegt.

11. Verfahren nach Anspruch 10 **dadurch gekennzeichnet, dass** die primäre Energiequelle (13), die als Netzteil (13) ausgeführt ist, über einen Akkulader (14) einen Akkumulator (15), als die sekundäre Energiequelle (15), versorgt.

12. Verfahren nach Anspruch 10 oder 11 **dadurch gekennzeichnet, dass** ein Leistungskontroller (16) und eine Strommessung (17) zwischengeschaltet sind und die aktuell verbrauchte Leistung gemessen wird.

13. Verfahren nach zumindest einem der vorhergehenden Ansprüche **dadurch gekennzeichnet, dass** der von der Strommessung (17) gemessene Wert der verbrauchten Leistung durch den Komparator (18) ausgewertet wird.

14. Verfahren nach zumindest einem der vorhergehenden Ansprüche **dadurch gekennzeichnet, dass** der Komparator (18) eine integrierte Hysterese aufweist, wobei der Komparator immer dann auf die Versorgung durch die sekundäre Energiequelle (15) um-/ zuschaltet, wenn die verbrauchte Leistung oberhalb der im Komparator (18) eingestellten Schwelle liegt, zuzüglich einer Abfallzeit bei Hysterese.

15. Verfahren nach zumindest einem der vorhergehenden Ansprüche **dadurch gekennzeichnet, dass** der Mikrocontroller (19) die Regelfunktion durch Ansteuerung des Akkuladers (24) und des Leistungskontrollers (26) übernimmt.

## Claims

1. A power circuit for a ventilator (1) with an electric motor (20) and a primary energy source (13) for supplying at least one power consumer (16, 17, 18, 19, 20, 21, 26, 27) and a secondary energy source (15) for at least temporarily supplying the power consumer (16, 17, 18, 19, 20, 21, 26, 27), **characterized in that** a currently consumed power is measured (16, 17) and evaluated (18, 19) and a comparator (18, 19) has a threshold value for the currently consumed power, wherein the comparator (18, 19), depending on the threshold value being reached, signals a power switch control (16, 26) to take the power for supplying the power consumer (16, 17, 18, 19, 20, 21, 26, 27) at least temporarily from the secondary energy source (15), wherein the supply by the secondary energy source is always switched/connected to when the consumed power is above the threshold set in the comparator (18, 19).

2. The power circuit according to claim 1, **characterized in that** the primary energy source (13), which is designed as a power supply unit (13), supplies an accumulator (15) as the secondary energy source (15) via an accumulator charger (14).

3. The power circuit according to claim 1 or 2, **characterized in that** a power switch control (16) and a current measurement unit (17) are interposed and the currently consumed power is measured.

4. The power circuit according to at least one of the preceding claims, **characterized in that** the value of the consumed power measured by the current measurement unit (17) is evaluated by the comparator (18).

5. The power circuit according to at least one of the preceding claims, **characterized in that** the comparator (18) has an integrated hysteresis, wherein the comparator always switches/connects to the supply by the secondary energy source (15) when the consumed power is above the threshold set in the comparator (18) plus a decay time in the case of hysteresis.

6. The power circuit according to at least one of the preceding claims, **characterized in that** the comparator (18) switches an accumulator charger (14) on and off.

7. The power circuit according to at least one of the preceding claims, **characterized in that** a connection (22) is additionally provided that, in addition or as an alternative to the primary energy source (13) and the secondary energy source (15), supplies the power consumers and the ventilator (1) in the event of mobile use of the ventilator.

8. The power circuit according to at least one of the preceding claims, **characterized in that** a micro-controller (19) takes over the regulating function by actuating the accumulator charger (24) and the power switch control (26).

9. The power circuit according to at least one of the preceding claims, **characterized in that** the circuit is realized in hardware and is additionally realized in firmware.

10. A method for power switching in a ventilator (1) with an electric motor (20) and a primary energy source (13) for supplying at least one power consumer (16, 17, 18, 19, 20, 21, 26, 27) and a secondary energy source (15) for at least temporarily supplying the power consumer (16, 17, 18, 19, 20, 21, 26, 27), **characterized in that** a currently consumed power is measured (16, 17) and evaluated (18, 19) and a comparator (18, 19) has a threshold value for the currently consumed power, wherein the comparator (18, 19), depending on the threshold value being reached, signals a power switch control (16, 26) to take the power for supplying the power consumer (16, 17, 18, 19, 20, 21, 26, 27) at least temporarily from the secondary energy source (15), wherein the supply by the secondary energy source is always switched/connected to when the consumed power is above the threshold set in the comparator (18, 19).

11. The method according to claim 10, **characterized in that** the primary energy source (13), which is designed as a power supply unit (13), supplies an accumulator (15) as the secondary energy source (15) via an accumulator charger (14).

12. The method according to claim 10 or 11, **characterized in that** a power switch control (16) and a current measurement unit (17) are interposed and the currently consumed power is measured.

13. The method according to at least one of the preceding claims, **characterized in that** the value of the consumed power measured by the current measurement unit (17) is evaluated by the comparator (18).

14. The method according to at least one of the preceding claims, **characterized in that** the comparator (18) has an integrated hysteresis, wherein the comparator always switches/connects to the supply by the secondary energy source (15) when the consumed power is above the threshold set in the comparator (18) plus a decay time in the case of hysteresis.

15. The method according to at least one of the preceding claims, **characterized in that** the micro-controller (19) takes over the regulating function by actuating the accumulator charger (24) and the power switch control (26).

## Revendications

1. Circuit de puissance pour un appareil respiratoire (1) avec un moteur électrique (20) et une source d'énergie primaire (13) pour l'alimentation d'au moins un consommateur électrique (16, 17, 18, 19, 20, 21, 26, 27) et une source d'énergie secondaire (15) pour l'alimentation au moins temporaire du consommateur électrique (16, 17, 18, 19, 20, 21, 26, 27), **caractérisé en ce qu'**une puissance consommée actuellement est mesurée (16, 17) et évaluée (18, 19), et un comparateur (18, 19) présente une valeur seuil pour la puissance consommée actuellement, le comparateur (18, 19) signalant à un contrôleur de puissance (16, 26), en fonction de l'atteinte de la valeur seuil, de prélever la puissance destinée à alimenter le consommateur électrique (16, 17, 18, 19, 20, 21, 26, 27) au moins partiellement à partir de la source d'énergie secondaire (15), la commutation sur la source d'énergie secondaire étant toujours effectuée lorsque la puissance consommée dépasse le seuil réglé dans le comparateur (18, 19).

2. Circuit de puissance selon la revendication 1, **caractérisé en ce que** la source d'énergie primaire (13), laquelle est conçue comme un bloc d'alimentation (13), alimente une batterie (15) comme source d'énergie secondaire (15) par le biais d'un chargeur de batterie (14).

3. Circuit de puissance selon la revendication 1 ou 2, **caractérisé en ce qu'**un contrôleur de puissance (16) et un dispositif de mesure de courant (17) sont intercalés et la puissance consommée actuellement est mesurée.

4. Circuit de puissance selon l'une au moins des revendications précédentes, **caractérisé en ce que** la valeur de la puissance consommée, mesurée par le dispositif de mesure de courant (17), est évaluée par le comparateur (18).

5. Circuit de puissance selon l'une au moins des revendications précédentes, **caractérisé en ce que** le comparateur (18) présente une hystérésis intégrée, dans lequel le comparateur commute toujours sur l'alimentation par la source d'énergie secondaire (15) lorsque la puissance consommée dépasse le seuil réglé dans le comparateur (18), en plus d'un temps de chute en cas d'hystérésis.

6. Circuit de puissance selon l'une au moins des revendications précédentes, **caractérisé en ce que** le comparateur (18) active et désactive un chargeur de batterie (Accu Charger, 14).

7. Circuit de puissance selon l'une au moins des revendications précédentes, **caractérisé en ce qu'**il est également prévu un raccordement (22), lequel alimente de façon complémentaire ou alternative à la source d'énergie primaire (13) et à la source d'énergie secondaire (15) les consommateurs électriques et l'appareil respiratoire (1) en cas d'utilisation mobile de l'appareil respiratoire.

8. Circuit de puissance selon l'une au moins des revendications précédentes, **caractérisé en ce qu'**un microcontrôleur (19) assure la fonction de réglage par activation du chargeur de batterie (24) et du contrôleur de puissance (26).

9. Circuit de puissance selon l'une au moins des revendications précédentes, **caractérisé en ce que** le circuit est réalisé sous forme de matériel, et également réalisé sous forme de micrologiciel.

10. Procédé de commutation de puissance pour un appareil respiratoire (1) avec un moteur électrique (20) et une source d'énergie primaire (13) destinée à alimenter au moins un consommateur électrique (16, 17, 18, 19, 20, 21, 26, 27) et une source d'énergie secondaire (15) destinée à alimenter au moins temporairement le consommateur électrique (16, 17, 18, 19, 20, 21, 26, 27), **caractérisé en ce qu'**une puissance consommée actuellement est mesurée (16, 17) et évaluée (18, 19), et un comparateur (18, 19) présente une valeur seuil pour la puissance consommée actuellement, dans lequel le comparateur (18, 19) signale à un contrôleur de puissance (16, 26), en fonction de l'atteinte de la valeur seuil, de prélever la puissance destinée à alimenter le consommateur électrique (16, 17, 18, 19, 20, 21, 26, 27) au moins partiellement à partir de la source d'énergie secondaire (15), la commutation sur la source d'énergie secondaire étant toujours effectuée lorsque la puissance consommée dépasse le seuil réglé dans le comparateur (18, 19).

11. Procédé selon la revendication 10, **caractérisé en ce que** la source d'énergie primaire (13), laquelle est conçue comme un bloc d'alimentation (13), alimente une batterie (15) comme source d'énergie secondaire (15) par le biais d'un chargeur de batterie (14).

12. Procédé selon la revendication 10 ou 11, **caractérisé en ce que** qu'un contrôleur de puissance (16) et un dispositif de mesure de courant (17) sont intercalés et la puissance consommée actuellement est mesurée.

13. Procédé selon l'une au moins des revendications précédentes, **caractérisé en ce que** la valeur de la puissance consommée, mesurée par le dispositif de mesure de courant (17), est évaluée par le comparateur (18).

14. Procédé selon l'une au moins des revendications précédentes, **caractérisé en ce que** le comparateur (18) présente une hystérésis intégrée, dans lequel le comparateur commute toujours sur l'alimentation par la source d'énergie secondaire (15) lorsque la puissance consommée dépasse le seuil réglé dans le comparateur (18), en plus d'un temps de chute en cas d'hystérésis.

15. Procédé selon l'une au moins des revendications précédentes, **caractérisé en ce que** le microcontrôleur (19) assure la fonction de réglage par activation du chargeur de batterie (24) et du contrôleur de puissance (26).
